Europäisches Patentamt

European Patent Office

Office européen des brevets

(11) Publication number: **0 272 745 B1**

# EUROPEAN PATENT SPECIFICATION

(45) Date of publication of patent specification: **22.04.92**  (51) Int. Cl.⁵: **A61K 6/06**

(21) Application number: **87202509.3**

(22) Date of filing: **14.12.87**

The file contains technical information submitted
after the application was filed and not included in
this specification

(54) **Translucent dental porcelain composition, its preparation and a restoration made thereof.**

(30) Priority: **23.12.86 US 945834**
**15.05.87 US 50011**
**01.12.87 US 124459**

(43) Date of publication of application:
**29.06.88 Bulletin 88/26**

(45) Publication of the grant of the patent:
**22.04.92 Bulletin 92/17**

(84) Designated Contracting States:
**AT BE CH DE ES FR GB GR IT LI LU NL SE**

(56) References cited:
**EP-A- 0 155 564          FR-A- 2 558 726**
**US-A- 3 052 083          US-A- 3 052 982**
**US-A- 4 101 330          US-A- 4 455 383**

**PERRY's CHEMICAL ENGINEERS' HAND-
BOOK, 6th ed.; p.21/15**

(73) Proprietor: **American Thermocraft Corp.**
**60 Franklin Street**
**East Orange New Jersey 07017(US)**

(72) Inventor: **Katz, Sigmund**
**24 Coolidge Avenue**
**East Orange, NJ 07052(US)**

(74) Representative: **Barendregt, Drs. F. et al**
**EXTERPATENT B.V. P.O. Box 90649**
**NL-2509 LP 's-Gravenhage(NL)**

EP 0 272 745 B1

Rank Xerox (UK) Business Services

## Description

FIELD OF THE INVENTION

The present invention relates to a dental porcelain composition.

Such compositions are known to comprise leucite ($K_2O.Al_2O_3.4SiO_2$) and are useful in the manufacture of porcelain dental restorations such as artificial teeth, crowns, bridges and the like.

BACKGROUND OF THE INVENTION

Porcelain is one of the most important materials used in dentistry. It lends itself to the manufacture of the most esthetic dental restorations since it can be colored to closely resemble the teeth it must replace.

Porcelain exhibits excellent chemical qualities insofar as dental applications are concerned. It is insoluble in the normal fluids of the oral cavity and in practically any given food or drink likely to pass through the oral cavity. It is also chemically able to resist the acid or alkali materials frequently used for washing artificial teeth. Moreover, mammalian tissues are very tolerant of its presence and such tolerance remains even after years of continuous contact.

Porcelain does have, however, one greast disadvantage. It is relatively fragile and repairs are difficult and costly. Because of the hazard of fragility, artificial dental crowns and bridges have heretofore been made using a metallic framework coated with a fused dental porcelain to provide the desired esthetics and strength.

The type of porcelain that is currently most often employed in dental restorations is typified by that described in the Weinstein et al patents, U.S. Patent Nos. 3,052,982 and 3,052,983. The Weinstein et al patents address the problem of preparing a porcelain whose coefficient of thermal expansion will match that of the metal base so that excessive stress formation will not occur during the production of the restoration.

The solution proposed by Weinstein et al was to make a dental porcelain composed of two different frits, one having a high coefficient of expansion and the other having a much lower coefficient of expansion, to result in a porcelain having a coefficient of expansion intermediate between the two materials, and which will match the dental alloy employed as the base.

The major disadvantage of the metal supported porcelain restoration is the loss of translucency which is especially noticeable at the gingival margin area. The junction of the restoration with the tooth may lack the translucent esthetic quality desired even in the case of an all-porcelain margin. Also, the added complexity of waxing, casting amd metal finishing requires an increased amount of labor for the production of the restoration. Still, it has been the most versatile restoration heretofore employed.

Mechanisms of strengthening ceramics, other than the use of metal ceramic systems, have primarily involved dispersion strengthening (aluminous core materials) and controlled crystallization (Dicor® castable glass ceramic from Dentsply International, Inc., York, Pennsylvania, and CeraPearl® castable glass ceramic from Kyocera, Inc., San Diego, California). The aforementioned systems are not greatly different in their clinical strength. Color is applied to the surface of such cast glasses, which limits their potential for optimum esthetics.

An alternative to the cast-glass ceramic systems is the extrusion molded system which employs an epoxy die and a shrink-free (expansion/contraction controlled) system, for example, Cerestore® nonshrink alumina ceramic of Johnson & Johnson Dental Products, Inc. The extrusion molded and cast-glass ceramic systems exhibit disadvantages, including a high initial equipment cost and the fact that each system is somewhat labor intensive.

A further disadvantage of systems employing an aluminous core is the reduced translucency produced by the semi-opaque nature of the core materials. Both the cast ceramic systems and the dispersion strengthened systems also have an inherent disadvantage in the manner in which they might be joined to form multiple units. Satisfactory commercially feasible systems of joining alumina reinforced ceramic units have not been developed.

Accordingly, it is an object of the present invention to provide a high strength dental porcelain for use in making all-ceramic dentures, crowns and bridges, thereby obviating the need for a metal or ceramic support.

A further object of the present invention is to provide a dental porcelain composition which is translucent and exhibits the ability to accept colors producing a restoration exhibiting desirable dental shades.

A still further object of this invention is to provide artifical crowns and bridges with greater impact strength and hence greater resistance to chipping.

A still further object is to provide a high strength dental porcelain composition which can be used employing present laboratory equipment and eliminating the need for extensive heat treatment.

A still further object is to provide permanent dental restorations exhibiting high strength, i.e., a minimum compressive strength of at least about $8.6 \times 10^5$ kPa (125,000 p.s.i.,) a diametral tensile strength of at least about $4.1 \times 10^4$ kPa (6,000 p.s.i.) and a flexural strength of at least about $1.1 \times 10^5$ kPa (16,000 p.s.i.)

## SUMMARY OF THE INVENTION

These as well as other objects and advantages can be achieved through the present invention which provides a translucent feldspathic dental porcelain composition useful for preparing dental restorations having a compressive strength of at least about $8.6 \times 10^5$ kPa (125,000 p.s.i), a diametral tensile strength of at least about $4.1 \times 10^4$ kPa 6,000 p.s.i), a flexural strength of at least about $1.1 \times 10^5$ kPa (16,000 p.s.i.) and a crystalline leucite content of at least 45% by weight, wherein said leucite crystallite exhibits a size of less than about 35 $\mu$m, preferably less than about 5 $\mu$m, comprising:

| Component | Percentage (by weight) |
|-----------|------------------------|
| $SiO_2$ | 55 - 70 |
| $Al_2O_3$ | 16 - 20 |
| CaO | 0.5 - 5.0 |
| MgO | 0.5 - 5.0 |
| $Li_2O$ | 1.0 - 5.0 |
| $Na_2O$ | 2.0 - 5.0 |
| $K_2O$ | 12.5 -22.5 |
| $Ce_2O_3$ | 0 - 1.0 |

## DETAILED DESCRIPTION OF THE INVENTION

The translucent feldspathic dental porcelain composition of the present invention can be made using a variety of feldspars, including Wyoming, Canadian, Norwegian, and Carolinian feldspars. These as well as other feldspars which have the following general composition are considered suitable for use in conjunction with the present invention:

| Component | Percentage (by weight) |
|-----------|------------------------|
| $SiO_2$ | 64 - 67 |
| $Al_2O_3$ | 17 - 20 |
| CaO | 0.5 - 1.0 |
| $K_2O$ | 12.0 - 14.0 |
| $Na_2O$ | 1.0 - 3.0 |

Preferably, Wyoming feldspar is used in making the dental porcelain of the present invention. At least a portion of the $K_2O$, $Al_2O_3$ and $SiO_2$ in such feldspars is currently believed to be present in a crystalline leucite configuration. Although not wishing to be bound by any theory or mechanism, it is currently believed that such leucite crystallites serve as nuclei during the fusing and cooling process in order to initiate further crystalline leucite nucleation and growth in the magma. As the magma is cooled, the crystalline leucite becomes less soluble and precipitates out.

$Na_2O$ is an inhibitor of leucite crystal growth during the fusing and cooling process. Low $Na_2O$ in conjunction with high $K_2O$ in the feldspar is believed to be responsible for the resulting high leucite content of the translucent feldspathic dental porcelain composition.

The feldspar is first culled to remove quartz, mica, and biotite. Next the feldspar is charged to a ball mill containing a grinding medium to reduce it to a fine powder, 95% of which passes through a 180 mesh (Tyler). screen. Then the feldspar is passed through a dry magnetic separator to remove any iron impurities that may be present. It is next further milled and screened through a 200 mesh (Tyler) screen.

The resultant powdered feldspar is blended with cerium oxide, if included, and a flux comprising any or all of the following: potassium nitrate, potassium silicate, lithium carbonate, calcium carbonate and magnesium oxide in quantities such that the resultant feldspathic dental porcelain, after fusing as hereinafter described, comprises:

| Component | Percentage (by weight) |
|---|---|
| $SiO_2$ | 55 - 70 |
| $Al_2O_3$ | 16 - 20 |
| CaO | 0.5 - 5.0 |
| MgO | 0.5 - 5.0 |
| $Li_2O$ | 1.0 - 5.0 |
| $Na_2O$ | 2.0 - 5.0 |
| $K_2O$ | 12.5 -22.5 |
| $Ce_2O_3$ | 0 - 1.0 |

Preferably, said resultant feldspathic dental porcelain composition comprises:

| Component | Percentage (by weight). |
|---|---|
| $SiO_2$ | 60 - 64 |
| $Al_2O_3$ | 16 - 19 |
| CaO | 0.5 - 2.0 |
| MgO | 0.5 - 1.5 |
| $Li_2O$ | 1.0 - 3.0 |
| $Na_2O$ | 2.0 - 4.0 |
| $K_2O$ | 12.5 -14.5 |
| $Ce_2O_3$ | 0 - 0.15 |

The quantity of flux needed will, of course, depend upon the particular composition of feldspar employed. Depending upon the initial fusing point of the feldspar, more or less flux will be needed in order that the fusing point is adjusted accordingly. For instance, a high fusing point feldspar will require more flux and a low fusing point feldspar, less flux.

The potassium oxide can be introduced by employing a combination of potassium nitrate and potassium silicate. It has surprisingly been found that this combination produces a much better product than either does alone.

From 2 to 7, preferably 3, wt % potassium nitrate can be used in the powdered dental porcelain composition of the present invention. The potassium nitrate functions to introduce potassium oxide into the silicate lattice, from which lattice the leucite crystals precipitate. The potassium oxide also lowers the fusing range.

From 3 to 10, preferably 5, wt % potassium silicate can be used in the powdered dental porcelain composition of the present invention. The potassium silicate functions in the same manner as the potassium nitrate, and the silicate tends to increase the silicate phase and to stabilize it such that leucite precipitation is more easily controlled resulting in the uniform distribution of leucite crystallites having a size of less than about 35 $\mu$m, preferably less than about 5 $\mu$m, throughout the glass matrix.

From 2.5 to 12.5, preferably from 2.5 to 7, and most preferably 3.5, wt % lithium carbonate can be used in the flux comprising the translucent feldspathic dental porcelain composition of the present invention. The lithium oxide is desired because it controls the fusing range without degrading other desirable properties. The incorporation of lithium oxide also functions to modify the viscosity during fusion so as to favor nucleation and crystalline leucite grain growth. The softer feldspars, for example, Carolinian feldspar, require less lithium carbonate than the harder feldspars, such as the Wyoming feldspar.

From 0.75 to 9, preferably from 0.75 to 5.5, and most preferably 2, wt% calcium carbonate can be used in the flux of the present invention. The calcium carbonate is desired because upon fusing it becomes calcium oxide which strengthens the glass phase and reduces its solubility in the presence of a high potassium oxide content.

From 0.5 to 5, preferably from 0.5 to 1.5, or most preferably 0.8, wt % magnesium oxide can be used in the translucent feldspathic dental porcelain composition of the present invention. The magnesium oxide is desired because it appears to function synergistically with the calcium oxide in strengthening the glass in relation to either alone.

From 0 to 1 wt %, preferably from 0 to 0.15 wt% cerium oxide can be used in the translucent feldspathic dental porcelain composition of the present invention. After fusion and cooling, without the incorporation of the cerium oxide, the resultant fused composition is extremely hard but can be milled by high impact comminution processes. Milling of such an extremely hard composition by attrition results in excessive fines and coarse particles, which are not useful since the resulting milled product cannot be wet. The cerium oxide in the composition of the present invention is desirable since it releases small amounts of oxygen at a point during the fusion where the viscosity is low enough such that bubbles are produced. The bubbles so created in the matrix allow for ready milling of the fused composition via both impact and attrition. The bubbles are also believed to provide extra surface for nucleation.

Nucleating agents, such as niobium oxide, can also be included in the translucent feldspathic dental porcelain composition in order to enhance leucite crystal formation. The addition of nucleating agents for such purposes is well known to those skilled in the art.

The unfired feldspathic mineral is opaque. Dental porcelain, of course, is highly translucent and is largely vitreous. By addition of potassium oxide and firing, most of the feldspathic mineral is converted to a vitreous phase.

The translucent feldspathic mixture, after blending, is charged into saggers and fused to form a vitreous body containing a uniform dispersion of leucite nuclei therein. The fusion can be carried out at about 1177°C (2150°F) to about 1288°F (2350°F,) preferably about 1232°C (2250°F,) for from about 2 to about 10, preferably about 5, hours. After the fusion, the porcelain composition is furnace-cooled at about 2,7°C (5°F)/min to about 1038°C (1900°F) held there for from about one to about four hours, and then quenched by immersion into water. The above fusion provides the requisite translucency, requisite crystalline leucite content, and desired leucite crystallite size of less than about 35 $\mu$m, preferably less than about 5 $\mu$m. The slow cooling to about 1038°C (1900°F) is essential for crystalline leucite nucleation and growth. Quenching at about 1038°C (1900°F) is also essential in order to arrest further growth of crystalline leucite such that the requisite translucency is provided. The quenched fused porcelain chunks are dried and then crushed and reduced to a fine powder by, for example, ball milling. Preferably the powder is fine enough to pass through a 180 to 200 mesh (Tyler) screen.

Since the feldspathic dental porcelain composition of the present invention is translucent, it is able to accept pigments and produce a restoration after firing exhibiting desirable dental shades. The usual pigments, such as chromates, vanadates, and manganates can be added to the feldspathic dental porcelain composition in small amounts, such as 0.5-1.5 weight percent, as well as opacifiers such as tin oxide, if desired. The thermal expansion of the porcelain is controlled to match that of the refractory die described below.

After the porcelain powder has been prepared and blended with the pigments, it is then employed in making dental restorations in the conventional manner; however, use of a metal or ceramic support is not required.

The general technique for the construction of a porcelain dental restoration (i.e. crown or bridge), is the following: first an impression is taken of the area that has been prepared to receive the dental restoration. A refractory die is prepared from the impression. The porcelain powder is then mixed with water to form a slurry, which is then applied to the refractory die by standard procedures.

Once the dental porcelain material is in its predetermined and desired shape, it is fired as is conventional for preparation of the various dental porcelain constructions in the art. The composition in its predetermined shape is first dried and then fired at a temperature and for a time such that the dental porcelain material fuses together as is conventional in the art for the preparation of a fired dental porcelain.

Typically, the composition of the present invention is fired at a temperature of from about 1024°C (1875°F) to about 1079°C (1975°F) preferably 1038°C (1900°F), for about 30 seconds. The furnace temperature is raised from about 538°C (1000°F) at the time of insertion to the desired temperature at a rate of from about 42°C (75°F) to about 69,4°C (125°F)/minute, preferably 56°C (100°F)/minute.

Once the composition has been fired, a dental restoration in the predetermined shape is provided, i.e., in the shape of a crown or bridge, for example, as discussed above.

By employing the composition of the present invention, the fused translucent feldspathic dental porcelain restoration thus obtained exhibits a compressive strength of at least about 8.6 x 10$^5$ kPa (125,000 p.s.i.), typically about 9,65 x 10$^5$ kPa (140,000 p.s.i); a diametral tensile strength of at least about 4,1 x 10$^4$ kPa (6,000 p.s.i)., typically about 6,9 x 10$^4$ kPa (10,000 p.s.i.); a flexural strength of at least about 1,1 x 10$^5$ kPa (16,000 p.s.i), typically about 1,4 x 10$^5$ kPa (20,000 p.s.i); and a crystalline leucite content of at least 45% by weight, typically 55 to 75% by weight; said leucite crystallites exhibiting a size of less than about 35 $\mu$m, preferably less than about 5 $\mu$m. These physical characteristics provide a dental composition with sufficient strength to obviate the need for a metal or ceramic support. Accordingly the practitioner can make the desired dental porcelain structure in one step.

The coefficient of thermal expansion of the high strength porcelain compositions of the present invention are significantly higher than the conventional porcelain compositions currently used for porcelain-fused-to-metal applications. As a result, the high strength porcelains of the present invention cannot be fused to the porcelain-fused-to-metal alloys currently available.

While the porcelain compositions of the present invention cannot be fused to any currently available porcelain-fused-to-metal alloys, they can be bonded to conventional dental metal substrates, if desired, using convention resin (filled or unfilled) bonding techniques.

The following examples are intended to illustrate, but not to limit, the present invention. Unlles otherwise stated, all percentages and parts are by weight.

EXAMPLE 1

Wyoming Feldspar, having the following composition:
65.3 wt % $SiO_2$
19.1 wt % $Al_2O_3$
 0.1 wt % CaO
 3.2 wt % $Na_2O$
12.1 wt % $K_2O$
 0.2 wt % *L.O.I.
*L.O.I. = Loss on ignition.
is culled to remove quartz, mica, and biotite. Next, the feldspar is charged into a ball mill containing a grinding medium and reduced to about 180 mesh (Tyler). Then, the feldspar is passed through a dry magnetic separator to remove any iron impurities. It may then be further milled for approximately 2 to 4 hours and screened through a 200 mesh screen. (Tyler)

The translucent feldspathic dental porcelain composition of the present invention was prepared by blending the components listed in Table I:

TABLE I

| Component | Grams |
|---|---|
| Wyoming Feldspar | 85.74 |
| $KNO_3$ | 3.00 |
| $K_2O \ SiO_2$ (1:2.5) | 5.00 |
| $Li_2CO_3$ | 3.50 |
| $CaCO_3$ | 1.90 |
| MgO | 0.76 |
| $Ce_2O_3$ | 0.10 |
| | 100.00 |

After weighing the raw materials, the components were blended, ball milled for 1 hour, transferred to a high alumina body sagger which was coated with a parting agent, for example, $Al_2O_3$, and heated to 1232°C (2250°F) (at a heat-up rate of 204°C (400°F)/hour) and maintained at that temperature for five hours. After fusion, the porcelain magma was cooled at about 2,7°C (5°)/min to 1038°C (1900°F) and held at 1038°C (1900°F) for from about 1 to about 4 hours. The magma was then quenched in water and dried. The porcelain composition so produced was crushed and ball milled such that the resulting particles pass through a 200 mesh (Tyler) screen.

The addition of pigments was accomplished by preparing a master batch at a pigment concentration of about 10% by weight. The master batch was prepared by the addition of the required pigment to a 1 kilogram batch of the translucent feldspathic dental porcelain composition and ball milling the mixture in order to disperse the pigments uniformly throughout.

To a 15 kilogram batch of the translucent feldspathic dental porcelain composition was added the following pigment master batch to produce a composition which, upon firing, resulted in a restoration with a desirable dental shade:

| Amount (grams) | Pigment (10% concentration) |
|---|---|
| 2.25 | Iron/Chrome/Zinc |
| 35.0 | Zirconium Praseodymium |
| 25.0 | Zirconium Vanadium Indium |
| 9.0 | Coral Brown |

The mixture was then ball milled to evenly disperse the pigments throughout.

EXAMPLE 2

The translucent feldspathic dental porcelain composition obtained from Example 1 and control 1 (Pencraft porcelain from American Thermocraft Corp., 60 Franklin Street, West Orange, New Jersey 07017) were formed into appropriate shapes for physical testing.

These structures were then dried at 538°C (100°F) for 6-8 minutes, followed by firing in an electric furnace to 1038°C (1900°F) (at a heat-up rate of 56°C (100°F)/min) and maintained at that temperature for 30 seconds and then allowed to cool to room temperature in air.

The following physical properties were measured for the translucent feldspathic dental porcelains so produced:

TABLE II

| | Example 2 | Control 1** |
|---|---|---|
| Compressive Strength kPa (p.s.i.) | $9,65 \times 10^5$ (140,000) | $3,4 \times 10^5$ (50,000) |
| Diametral Tensile Strength kPa (p.s.i.) | $8,2 \times 10^7$ (12,000) | $4,1 \times 10^4$ (6,000) |
| Flexural Strength kPa (p.s.i.) | $1,4 \times 10^5$ (20,000) | $7,5 \times 10^4$ (11,000) |
| *Leucite Content (% by weight) | Example 2 was about 40% greater than control 1. | |
| Coefficient of Thermal Expansion (in./in.°C) | $18 \times 10^{-6}$ | $13.2 \times 10^{-6}$ |

*Estimated by Xray Diffraction.
**Fired to its fully matured temperature of 982°C (1800°F.)

EXAMPLE 3

Preparation of a Dental Restoration of the Present Invention.

An impression was taken of the area that had been prepared to receive the dental restoration. A refractory die was prepared from the impression. The die was soaked in water so as not to absorb the water from the porcelain slurry when applied thereto. The feldspathic translucent dental porcelain composition of Example 1 was mixed with water to form a slurry. The slurry was applied to the die using a spatula and forming a rough facsimile of the desired dental restoration. Gingival porcelain was built first. Then incisal porcelain was blended over the gingival porcelain. The water was then removed by a combination of vibration and absorption with a tissue. The exact configuration of the desired restoration was then carved by a dental laboratory technician.

The unfired restoration was dried outside a furnace held at 538°C (1000°F) for 6-8 minutes. It was then placed in the furnace and the temperature raised to 1038°C (1900°F) and held for 30 seconds (at a heat-up rate of 56°C/min (100°F/min).) The restoration was then removed from the furnace and allowed to cool to room temperature in air. Appropriate porcelain additions were made in order to perfect the configuration of the restoration, and the restoration was refired as necessary. Between firings, adjustments were made with appropriate grinding instruments. In this manner, an all-porcelain dental restoration was obtained exhibiting sufficiently high strength such that a conventional metal or ceramic support was not necessary for the resulting restoration to meet all current dental requirements.

**Claims**

**1.** A dental porcelain composition, **characterized in that** said composition is a translucent feldspathic dental porcelain composition exhibiting a crystalline leucite content of at least 45% by weight wherein said leucite crystallites exhibit a size of less than about 35 $\mu$m, comprising:

| Component | Percentage (by weight) |
|---|---|
| $SiO_2$ | 55 - 70 |
| $Al_2O_3$ | 16 - 20 |
| CaO | 0.5- 5.0 |
| MgO | 0.5- 5.0 |
| $Li_2O$ | 1.0- 5.0 |
| $Na_2O$ | 2.0- 5.0 |
| $K_2O$ | 12.5- 22.5 |
| $Ce_2O_3$ | 0 - 1.0 |

2. A dental porcelain composition according to Claim 1, **characterized in that** the leucite crystallites exhibit a size of less than about 5 $\mu$m.

3. A dental porcelain composition according to Claim 1**, characterized in that** the feldspathic nature of the composition is imparted thereto by having incorporated therein a feldspar exhibiting a composition comprising:

| Component | Percentage (by weight) |
|---|---|
| $SiO_2$ | 64 - 67 |
| $Al_2O_3$ | 17 - 20 |
| CaO | 0.5- 1.0 |
| $K_2O$ | 12.0- 14.0 |
| $Na_2O$ | 1.0- 3.0 |

4. A dental porcelain composition according to Claim 3, **characterized that** at least a portion of the $K_2O$, $Al_2O_3$ and $SiO_2$ therein are present in a crystalline leucite configuration.

5. A dental porcelain composition according to Claim 4, **characterized in that** said feldspar is Wyoming feldspar.

6. A dental porcelain composition according to Claim 1, **characaterized in that** it comprises:

| Component | Percentage (by weight) |
|---|---|
| $SiO_2$ | 60 - 64 |
| $Al_2O_3$ | 16 - 19 |
| CaO | 0.5- 2.0 |
| MgO | 0.5- 1,5 |
| $Li_2O$ | 1.0- 3,0 |
| $Na_2O$ | 2.0- 4.0 |
| $K_2O$ | 12.5- 14.5 |
| $Ce_2O_3$ | 0 - 0.15 |

7. A dental porcelain composition according to Claim 1, **characaterized in that** it further comprises at least one pigment.

8. A dental porcelain composition according to Claim 6, **characterized in that** it further comprises at least one pigment.

**9.** A dental porcelain composition according to Claim 7, **characterized in that** said pigment is selected from the group consisting of chromates, vanadates, manganates and mixtures thereof.

**10.** A dental porcelain composition according to Claim 8, **characterized in that** said pigment is selected from the group consisting of chromates, vanadates, manganates, and mixtures thereof.

**11.** A method of preparing a dental composition **characterized in that** a composition exhibiting a crystalline leucite content of at least 45% by weight, wherein said leucite crystallites exhibit a size of less than about 35 $\mu$m is obtained in a process comprising the steps of:

a) culling a feldspar having the composition comprising:

| Component | Percentage (by weight) |
|---|---|
| $SiO_2$ | 64 - 67 |
| $Al_2O_3$ | 17 - 20 |
| $CaO$ | 0.5- 1.0 |
| $K_2O$ | 12.0- 14.0 |
| $Na_2O$ | 1.0- 3.0 |

to remove quartz, mica and biotite;

b) grinding said feldspar;

c) passing said ground feldspar through a 200 mesh screen;

d) blending the screened feldspar with potassium nitrate, potassium silicate, lithium carbonate, calcium carbonate, magnesium oxide, cerium oxide, alumina and/or aluminium silicate in quantities such that the resultant feldspathic dental porcelain composition, after fusion, comprises:

| Component | Percentage (by weight) |
|---|---|
| $SiO_2$ | 55 - 70 |
| $Al_2O_3$ | 16 - 20 |
| $CaO$ | 0.5- 5.0 |
| $MgO$ | 0.5- 5.0 |
| $Li_2O$ | 1.0- 5.0 |
| $Na_2O$ | 2.0- 5.0 |
| $K_2O$ | 12.5- 22.5 |
| $Ce_2O_3$ | 0 - 1.0 |

e) fusing said feldspathic dental porcelain composition at from about 1177°C (2150°F) to about 1288°C (2350°F) for from about 2 to about 10 hours at a heat-up rate of about 204°C (400°F)/hour, thereby forming a magma;

f) cooling said magma at about 2,7°C (5°F)/minute to about 1038°C (1900°F);

g) holding said cooled magma at about 1038°C (1900°F) for from about one to about four hours;

h) quenching said cooled magma in water; and

i) milling said quenched magma such that 95% thereof passes through a 180 mesh (Tyler) screen, thereby forming said translucent feldspathic dental porcelain composition.

**12.** A method according to Claim 11, **characterized in that** at least a portion of the $K_2O$, $Al_2O_3$ and $SiO_2$ in said feldspar is present in a crystalline leucite configuration.

**13.** A method according to Claim 12, **characterized in that** said feldspar is Wyoming feldspar.

**14.** A method according to Claim 11, **characterized in that** it further comprises the step of passing said ground feldspar through a dry magnetic separator to remove iron after grinding.

**15.** A method according to Claim 11, **characterized that** the fusing temperature is about 1232°C (2250°F).

**16.** A method according to Claim 15, **characterized in that** the fusing temperature is maintained for about 5 hours.

**17.** A dental porcelain restoration, **characterized in that** it exhibits a minimum crystalline leucite content of 45% by weight, wherein said leucite crystallites exhibit a size of less than about 35 $\mu$m; a compressive strength of at least about 8,6 x $10^5$ kPa (125,000 p.s.i.); a diametral tensile strength of at least about 4,1 x $10^4$ kPa (6,000 p.s.i.); and a flexural strength of at least about 1,1 x $10^5$ kPa (16,000 p.s.i.); comprising:

| Component | Percentage (by weight) |
|---|---|
| $SiO_2$ | 55 - 70 |
| $Al_2O_3$ | 16 - 20 |
| CaO | 0.5- 5.0 |
| MgO | 0.5- 5.0 |
| $Li_2O$ | 1.0- 5.0 |
| $Na_2O$ | 2.0- 5.0 |
| $K_2O$ | 12.5- 22.5 |
| $Ce_2O_3$ | 0 - 1.0 |

**18.** A dental porcelain restoration according to Claim 17, **characterized in that** the leucite crystallites exhibit a size of less than about 5 $\mu$m.

**19.** A dental porcelain restoration according to Claim 17, **characterized in that** it comprises:

| Component | Percentage (by weight) |
|---|---|
| $SiO_2$ | 60 - 64 |
| $Al_2O_3$ | 16 - 19 |
| CaO | 0.5- 2.0 |
| MgO | 0.5- 1.5 |
| $Li_2O$ | 1.0- 3.0 |
| $Na_2O$ | 2.0- 4.0 |
| $K_2O$ | 12.5- 14.5 |
| $Ce_2O_3$ | 0 - 0.15 |

**20.** A dental porcelain restoration according to Claim 17, **characterized in that** it comprises at least one pigment.

**21.** A dental porcelain restoration according to Claim 19, **characterized in that** it further comprises at least one pigment.

**22.** A dental porcelain restoration according to Claim 20, **characterized in that** said pigment is selected from the group consisting of chromates, vanadates, manganates, and mixtures thereof.

**Revendications**

**1.** Une composition de porcelaine dentaire, caractérisée en ce que ladite composition est une composition de porcelaine dentaire feldspathique translucide présentant une teneur en leucite cristalline d'au moins 45% en poids, dans laquelle lesdites cristallites de leucite présentent une dimension inférieure à environ 35 $\mu$m, comprenant:

EP 0 272 745 B1

| Composant | % (en poids) |
|---|---|
| $SiO_2$ | 55 - 70 |
| $Al_2O_3$ | 16 - 20 |
| CaO | 0,5 - 5,0 |
| MgO | 0,5 - 5,0 |
| $Li_2O$ | 1,0 - 5,0 |
| $Na_2O$ | 2,0 - 5,0 |
| $K_2O$ | 12,5 - 22,5 |
| $Ce_2O_3$ | 0 - 1,0 |

2. Une composition de porcelaire dentaire selon la revendication 1, caractérisée en ce que les cristallites de leucite présentent une dimension inférieure à environ 5 $\mu$m.

3. Une composition de porcelaine dentaire selon la revendication 1, caractérisée en ce que la nature feldspathique de la composition lui est impartie par incorporation à ladite composition d'un feldspath présentant une composition à base de:

| Composant | % (en poids) |
|---|---|
| $SiO_2$ | 64 - 67 |
| $Al_2O_3$ | 17 - 20 |
| CaO | 0,5 - 1,0 |
| $K_2O$ | 12,0 - 14,0 |
| $Na_2O$ | 1,0 - 3,0 |

4. Une composition de porcelaine dentaire selon la revendication 3, caractérisée en ce qu'au moins une partie desdits $K_2O$, $Al_2O_3$ et $SiO_2$ sont présents dans une configuration de la leucite cristalline.

5. Une composition de porcelaine dentaire selon la revendication 4, caractérisée en ce que ledit feldspath est du feldspath Wyoming.

6. Une composition de porcelaine dentaire selon la revendication 1, caractérisée en ce qu'elle comprend:

| Composant | % (en poids) |
|---|---|
| $SiO_2$ | 60 - 64 |
| $Al_2O_3$ | 16 - 19 |
| CaO | 0,5 - 2,0 |
| MgO | 0,5 - 1,5 |
| $Li_2O$ | 1,0 - 3,0 |
| $Na_2O$ | 2,0 - 4,0 |
| $K_2O$ | 12,5 - 14,5 |
| $Ce_2O_3$ | 0 - 0,15 |

7. Une composition de porcelaine dentaire selon la revendication 1, caractérisée en ce qu'elle comprend de plus au moins un pigment.

8. Une composition de porcelaine dentaire selon la revendication 6, caractérisée en ce qu'elle comprend en outre au moins un pigment.

9. Une composition de porcelaine dentaire selon la revendication 7, caractérisée en ce que ledit pigment est choisi parmi le groupe consistant en chromates, vanadates, manganates et leurs mélanges.

**10.** Une composition de porcelaine dentaire selon la revendication 8, caractérisée en ce que ledit pigment est choisi parmi le groupe consistant en chromates, vanadates, manganates et leurs mélanges.

**11.** Un procédé de préparation d'une composition dentaire caractérisé en ce qu'une composition présentant une teneur en leucite cristalline d'au moins 45% en poids, dans laquelle lesdites cristallites de leucite présentent une dimension inférieure à environ 35 $\mu$m, est obtenue dans un procédé comprenant en les étapes de:

a) épierrage d'un feldspath présentant la composition comprenant:

| Composant | % (en poids) |
|---|---|
| $SiO_2$ | 64 - 67 |
| $Al_2O_3$ | 17 - 20 |
| CaO | 0,5 - 1,0 |
| $K_2O$ | 12,0 - 14,0 |
| $Na_2O$ | 1,0 - 3,0 |

pour enlever le quartz, le mica et la biotite;

b) broyage dudit feldspath;

c) passage dudit feldspath moulu dans un tamis de 200 mesh;

d) mélange du feldspath tamisé avec du nitrate de potassium, du silicate de potassium, du carbonate de lithium, du carbonate de calcium, de l'oxyde de magnésium, de l'oxyde de cérium, de l'alumine et/ou du silicate d'aluminium en quantités telles que la composition de porcelaine dentaire feldspathique qui en résulte comprenne, après fusion:

| Composant | % (en poids) |
|---|---|
| $SiO_2$ | 55 - 70 |
| $Al_2O_3$ | 16 - 20 |
| CaO | 0,5 - 5,0 |
| MgO | 0,5 - 5,0 |
| $Li_2O$ | 1,0 - 5,0 |
| $Na_2O$ | 2,0 - 5,0 |
| $K_2O$ | 12,5 - 22,5 |
| $Ce_2O_3$ | 0 - 1,0 |

e) fusion de ladite composition de porcelaine dentaire feldspathique à partir d'environ 1 177°C (2 150°F) jusqu'à environ 1 288°C (2 350°F) pour une durée d'environ 2 à environ 10 heures à un taux de chauffage d'environ 204°C/heure (400°F), formant par là un magma;

f) refroidissement dudit magma à environ 2,7°C/minute (5°F) à environ 1 038°C (1 900°F);

g) maintien dudit magma refroidi à environ 1 038°C (1 900°F) pendant une durée d'environ 1 à environ 4 heures;

h) trempe dudit magma refroidi dans l'eau; et

i) broyage dudit magma trempé de telle sorte que 95% de ce magma passent à travers un tamis Tyler de 180 mesh, formant par là même ladite composition de porcelaine dentaire feldspathique translucide.

**12.** Un procédé selon la revendication 11, caractérisé en ce qu'au moins une partie desdits $K_2O$, Al$_2$O3 et $SiO_2$ dans ledit feldspath est présente dans la configuration à base de leucite cristalline.

**13.** Un procédé selon la revendication 12, caractérisé en ce que ledit feldspath est du feldspath Wyoming.

**14.** Un procédé selon la revendication 11, caractérisé en ce qu'il comprend de plus l'étape de passage dudit feldspath moulu à travers un séparateur magnétique sec afin d'en retirer le fer après broyage.

**15.** Un procédé selon la revendication 11, caractérisé en ce que la température de fusion est de l'ordre de 1 232°C (2 250°F).

**16.** Un procédé selon la revendication 15, caractérisé en ce que la température de fusion est maintenue pendant environ 5 heures.

**17.** Une restauration à base de porcelaine dentaire, caractérisée en ce qu'elle présente un minimum de teneur en leucite cristalline de 45% en poids, dans laquelle lesdites cristallites de leucite présentent une dimension inférieure à environ 35 $\mu$m; une résistance à la compression d'au moins environ 8,6 x $10^5$ kPa (125 000 psi); une résistance à la rupture par traction d'au moins environ 4,1 x $10^4$ kPa (6 000 psi); et une raideur à la flexion d'au moins environ 1,1 x $10^5$ kPa (16 000 psi); comprenant:

| Composant | % (en poids) |
|---|---|
| $SiO_2$ | 55 - 70 |
| $Al_2O_3$ | 16 - 20 |
| $CaO$ | 0,5 - 5,0 |
| $MgO$ | 0,5 - 5,0 |
| $Li_2O$ | 1,0 - 5,0 |
| $Na_2O$ | 2,0 - 5,0 |
| $K_2O$ | 12,5 - 22,5 |
| $Ce_2O_3$ | 0 - 1,0 |

**18.** Une restauration en porcelaine dentaire selon la revendication 17, caractérisée en ce que les cristallites de leucite présentent une dimension inférieure à environ 5 $\mu$m.

**19.** Une restauration en porcelaine dentaire selon la revendication 17, caractérisée en ce qu'elle comprend:

| Composant | % (en poids) |
|---|---|
| $SiO_2$ | 60 - 64 |
| $Al_2O_3$ | 16 - 19 |
| $CaO$ | 0,5 - 2,0 |
| $MgO$ | 0,5 - 1,5 |
| $Li_2O$ | 1,0 - 3,0 |
| $Na_2O$ | 2,0 - 4,0 |
| $K_2O$ | 12,5 - 14,5 |
| $Ce_2O_3$ | 0 - 0,15 |

**20.** Une restauration en porcelaine dentaire selon la revendication 17, caractérisée en ce qu'elle comprend au moins un pigment.

**21.** Une restauration en porcelaine dentaire selon la revendication 19, caractérisée en ce qu'elle comprend en outre au moins un pigment.

**22.** Une restauration en porcelaine dentaire selon la revendication 20, caractérisée en ce que ledit pigment est choisi parmi le groupe consistant en chromates, vanadates, manganates et leurs mélanges.

**Patentansprüche**

**1.** Dentalporzellanzusammensetzung
dadurch **gekennzeichnet,** daß
es sich bei der Zusammensetzung um eine durchscheinende Dentalporzellanzsusammensetzung auf feldspathaltiger Basis handelt, das einen Anteil von mindestens 45 Gew.&% an kristallinem Leuzit enthält, wobei die Leuzitkristallite eine Größe von weniger als rund 35 $\mu$m aufweisen, die folgende Anteile enthält:

EP 0 272 745 B1

| Bestandteil | Anteil (Gew.%) |
|---|---|
| $SiO_2$ | 55 - 70 |
| $Al_2O_3$ | 16 - 20 |
| $CaO$ | 0.5 - 5.0 |
| $MgO$ | 0.5 - 5.0 |
| $Li_2O$ | 1.0 - 5.0 |
| $Na_2O$ | 2.0 - 5.0 |
| $K_2O$ | 12.5 - 22.5 |
| $Ce_2O_3$ | 0 - 1.0 |

2. Dentalporzellanzusammensetzung nach Anspruch 1,
dadurch **gekennzeichnet,** daß
die Leuzitkristallite eine Größe von weniger als rund 5 $\mu$m aufweisen.

3. Dentalporzellanzusammensetzung nach Anspruch 1,
dadurch **gekennzeichnet,** daß
der Zusammensetzung ihr feldspatähnlicher Charakter dadurch verliehen wird, daß in ihr ein Feldspat eingearbeitet ist, der wie folgt zusammengesetzt ist:

| Bestandteil | Anteil (Gew.%) |
|---|---|
| $SiO_2$ | 64 - 67 |
| $Al_2O_3$ | 17 - 20 |
| $CaO$ | 0.5 - 1.0 |
| $K_2O$ | 12.0 - 14.0 |
| $Na_2O$ | 1.0 - 3.0 |

4. Dentalporzellanzusammensetzung nach Anspruch 3,
dadurch **gekennzeichnet,** daß
die Zusammensetzungsanteile von $K_2O$, $Al_2O_3$ und $SiO_2$ zumindest teilweise in kristalliner Leuzitform vorliegen.

5. Dentalporzellanzusammensetzung nach Anspruch 4,
dadurch **gekennzeichnet,** daß
es sich bei dem Feldspat um Wyoming-Feldspat handelt.

6. Dentalporzellanzusammensetzung nach Anspruch 1,
dadurch **gekennzeichnet,** daß
sie folgende Anteile enthält:

| Bestandteil | Anteil (Gew.%) |
|---|---|
| $SiO_2$ | 60 - 64 |
| $Al_2O_3$ | 16 - 19 |
| $CaO$ | 0.5 - 2.0 |
| $MgO$ | 0.5 - 1.5 |
| $Li_2O$ | 1.0 - 3.0 |
| $Na_2O$ | 2.0 - 4.0 |
| $K_2O$ | 12.5 - 14.5 |
| $Ce_2O_3$ | 0 - 0.15 |

7. Dentalporzellanzusammensetzung nach Anspruch 1,
dadurch **gekennzeichnet,** daß

14

sie außerdem mindestens einen Farbstoff enthält.

8. Dentalporzellanzusammensetzung nach Anspruch 6,
dadurch **gekennzeichnet,** daß
sie außerdem mindestens einen Farbstoff enthält.

9. Dentalporzellanzusammensetzung nach Anspruch 7,
dadurch **gekennzeichnet,** daß
der Farbstoff aus der Gruppe gewählt wird, die Chromate, Vanadate, Manganate und deren Gemische
umfaßt.

10. Dentalporzellanzusammensetzung nach Anspruch 8,
dadurch **gekennzeichnet,** daß
der Farbstoff aus der Gruppe gewählt wird, die Chromate, Vanadate, Manganate und deren Gemische
umfaßt.

11. Verfahren zur Herstellung einer Dentalporzellanzusammensetzung,
dadurch **gekennzeichnet,** daß
eine Zusammensetzung mit einem Anteil an kristallinem Leuzit von mindestens 45 Gew.%, wobei die
Leuzitkristallite eine Größe von weniger als etwa 35 um aufweisen, in einem Verfahren hergestellt wird,
das die folgenden Schritte aufweist:
a) Ausklauben eines Feldspats mit folgender Zusammensetzung

| Bestandteil | Anteil (Gew.%) |
|---|---|
| $SiO_2$ | 64 - 67 |
| $Al_2O_3$ | 17 - 20 |
| $CaO$ | 0.5 - 1.0 |
| $K_2O$ | 12.0 - 14.0 |
| $Na_2O$ | 1.0 - 3.0 |

um Quarz, Glimmer und Biotit zu entfernen;
b) Vermahlen des Feldspats;
c) Durchsieben des gemahlenen Feldspats durch ein Maschensieb mit einer Maschenweite von 200;
d) Versetzen des durchgesiebten Feldspats mit Kaliumnitrat, Kaliumsilikat, Lithiumkarbonat, Kalziumkarbonat, Magnesiumoxid, Ceroxid, Tonerde- und/oder Aluminiumsilikat jeweils in einer Menge, daß
das nach einem Schmelzvorgang vorliegende feldspathaltige Dentalporzellanzusammensetzung
folgende Anteile aufweist:

| Bestandteil | Anteil (Gew.%) |
|---|---|
| $SiO_2$ | 55 - 70 |
| $Al_2O_3$ | 16 - 20 |
| $CaO$ | 0.5 - 5.0 |
| $MgO$ | 0.5 - 5.0 |
| $Li_2O$ | 1.0 - 5.0 |
| $Na_2O$ | 2.0 - 5.0 |
| $K_2O$ | 12.5 - 22.5 |
| $Ce_2O_3$ | 0 - 1.0 |

e) zur Bildung eines Magmas Aufschmelzen der feldspathaltigen Dentalporzellanzusammensetzung
bei einer Temperatur zwischen rund 1177 °C (2150 °F) und rund 1288 °C (2350 °F) über einen
Zeitraum von rund 2 bis rund 10 Stunden bei einer Erwärmungsgeschwindigkeit von rund 204 °C
(400 °F)/Std.;
f) Herunterkühlen des Magmas mit einer Geschwindigkeit von rund 2,7 °C (5 °F)/Minute auf einen
Wert von rund 1038 °C (1900 °F);
g) Halten des heruntergekühlten Magmas auf einem Temperaturwert von rund 1038 °C /1900 °F) über einen Zeitraum von etwa einer Stunde bis rund vier Stunden;

h) Abschrecken des heruntergekühlten Magmas In Wasser; und

i) Vermahlen des abgeschreckten Magmas In der Weise, daß 95% seiner Masse durch ein Maschensieb (Tyler-Sieb von Maschenweite 180 hindurchgehen, um so die durchscheinende feldspathaltige Dentalporzellanzusammensetzung zu erhalten.

**12.** Verfahren nach Anspruch 11,
dadurch **gekennzeichnet,** daß
die Zusammensetzungsanteile $K_2O$, $Al_2O_3$ und $SiO_2$ zumindest teilweise in kristalliner Leuzitform in dem Feldspat vorliegen.

**13.** Verfahren nach Anspruch 12,
dadurch **gekennzeichnet,** daß
es sich bei dem Feldspat um Wyoming-Feldspat handelt.

**14.** Verfahren nach Anspruch 11,
dadurch **gekennzeichnet,** daß
es des weiteren einen Verfahrensschritt aufweist, bei dem zur Herauslösung von Eisen der vermahlene Feldspat einen Trockenmagnetabscheider durchläuft.

**15.** Verfahren nach Anspruch 11,
dadurch **gekennzeichnet,** daß
die Schmelztemperatur rund 1232 ° C (2250 ° F) beträgt.

**16.** Verfahren nach Anspruch 15,
dadurch **gekennzeichnet,** daß
die Schmelztemperatur rund 5 Stunden lang aufrechterhalten wird.

**17.** Dentalporzellan-Restauration
dadurch **gekennzeichnet,** daß
sie einen Anteil an kristallinem Leuzit von mindestens 45 Gew.% aufweist, wobei die Leuzitkristallite eine Größe von weniger als rund 35 $\mu$m aufweisen; daß ihre Druckfestigkeit mindestens bei etwa 8,5 x $10^5$ kPa (125.000 p.s.i.) beträgt; daß sie eine diametrale Zugfestigkeit von mindestens rund 4,1 x $10^4$ kPa (6.000 p.s.i.) aufweist, sowie eine Biegefestigkeit von mindestens rund 1,1 x $10^5$ kPa (16.000 p.s.i.), und folgende Zusammensetzung hat:

| Bestandteil | Anteil (Gew.%) |
|---|---|
| $SiO_2$ | 55 - 70 |
| $Al_2O_3$ | 16 - 20 |
| CaO | 0.5 - 5.0 |
| MgO | 0.5 - 5.0 |
| $Li_2O$ | 1.0 - 5.0 |
| $Na_2O$ | 2.0 - 5.0 |
| $K_2O$ | 12.5 - 22.5 |
| $Ce_2O_3$ | 0 - 1.0 |

**18.** Dentalporzellan-Restauration nach Anspruch 17,
dadurch **gekennzeichnet,** daß
die Leuzitkristallite eine Größe von Weniger als etwa 5 $\mu$m aufweisen.

**19.** Dentalporzellan-Restauration nach Anspruch 17,
dadurch **gekennzeichnet,** daß
sie folgende Zusammensetzung aufweist:

| | |
|---|---|
| $SiO_2$ | 60 - 64 |
| $Al_2O_3$ | 16 - 19 |
| CaO | 0.5 - 2.0 |
| MgO | 0.5 - 1.5 |
| $Li_2O$ | 1.0 - 3.0 |
| $Na_2O$ | 2.0 - 4.0 |
| $K_2O$ | 12.5 - 14.5 |
| $Ce_2O_3$ | 0 - 0.15 |

**20.** Dentalporzellan-Restauration nach Anspruch 17,
dadurch **gekennzeichnet,** daß
sie mindestens einen Farbstoff enthält.

**21.** Dentalporzellan-Restauration nach Anspruch 19,
dadurch **gekennzeichnet,** daß
sie zusätzlich mindestens einen Farbstoff enthält.

**22.** Dentalporzellan-Restauration nach Anspruch 20,
dadurch **gekennzeichnet,** daß
der Farbstoff aus der Gruppe gewählt wird, die Chromate, Vanadate, Manganate und deren Gemische umfaßt.